# EUROPEAN PATENT APPLICATION

(11) **EP 3 705 035 A1**
(43) Date of publication of application: **09.09.2020**
(21) Application number: 19161223.3
(22) Date of filing: 07.03.2019
(51) Int. Cl.: A61B 5/0408, A61B 5/00

(54) **MANUFACTURING OF SKIN-COMPATIBLE ELECTRODES**

(71) Applicant: Nederlandse Organisatie voor toegepast- natuurwetenschappelijk Onderzoek TNO, 2595 DA 's-Gravenhage (NL); Henkel AG & Co. KGaA, 40589 Düsseldorf (DE)
(72) Inventor: ZALAR, Peter, 2595 DA 's-Gravenhage (NL); SMITS, Edsger Constant Pieter, 2595 DA 's-Gravenhage (NL); VAN DER MEULEN, Inge, 5521 WM Eersel (NL); GILLISSEN, Stijn, 3520 Zonhoven (BE); NEGELE, Carla, 41472 Neuss (DE); GOETHEL, Frank, 09125 Chemnitz (DE); ROSCHEK, Tobias, 42699 Solingen (DE); BESLER, Alissa, 40591 Düsseldorf (DE)
(74) Representative: V.O.

(57) **Abstract**

A method of manufacturing a skin-compatible electrode (100) comprises printing a circuit pattern (P1) onto a flexible substrate (200) to form an electrically conductive pattern including an electrode pad area (301). A layer of an adhesive composition (401p) is printed in a second pattern (P2) onto the electrode pad area (301) to form an adhesive interface layer (401). The adhesive interface layer (401) is a dry film formed from the adhesive composition (401p) comprising an ionically conductive pressure sensitive adhesive composition comprising a resin (R), an ionic liquid (I), and optionally electrically conductive particles (P). A layer thickness and material of the flexible substrate, the conductive pattern, and the conductive adhesive interface have relatively low stiffness in plane of the flexible substrate (200).

## Description

### TECHNICAL FIELD AND BACKGROUND

The present invention relates to a method for the production of ExG electrodes and patches for application to human subjects.

Bio potential electrodes are used to measure bio signals such as electrocardiography (ECG), electroencephalography (EEG) and electromyography (EMG).

For example, currently used ECG electrodes are connected to the skin via gel, which acts as an electrolyte and couples the electrical potential in the body to the electrode. However, presently used electrodes typically dry out over time and cannot be used for prolonged measurements. Most of the presently used electrodes are not recommended for use longer than 24h. In addition, they do not have long storage times in air. Most of the presently used electrodes expire within one month after opening the hermetic packaging preventing them from drying out during storage.

Currently used gel electrodes comprise high salt concentrations, which are needed for providing low impedances and good signal quality, however at the same time they cause skin irritation with many patients. Furthermore, presently used electrodes which are based on a hydrogel contain relatively high quantities of water. The high water content is the reason why these electrodes tend to dry out over time. Presently used electrodes which are based on ionic conductivity can therefore not be used for long-term measurements (e.g. three days) since the signal quality decreases along with decreasing water content. Current gel electrodes are attached to the skin with a ring of a pressure sensitive skin adhesive surrounding the inner gel.

There are also tab electrodes currently on the market, which are attached to the skin via a gel-type adhesive. These electrodes do not need an additional skin adhesive, since the gel itself is adhering to the skin. However, these electrodes also comprise a salt and water, and dry out over time and are therefore not suitable for prolonged measurements. The cohesion of the adhesive is often poor in these electrodes, which for example leads to cohesive failure upon removal of the electrode. Furthermore, production of such electrodes is laborious due to the difficult handling of the adhesive, e.g. placement of the gel film atop the conductive bottom layers.

Alternatively, a pressure sensitive adhesive comprising conductive fillers, such as carbon black can be used in the electrodes to measure bio signals. The drawback in this kind of electrodes is that a high carbon black concentration is needed, which leads to reduced adhesion properties. Furthermore, the signal quality in this kind of electrodes is poor.

In another electrode solution, the electrode comprises adhesives comprising the combination of carbon black and a salt. An electrophoretic alignment of conductive fillers is required in order to obtain sufficient impedances in this solution. However, this electrophoretic activation step makes the electrode production expensive and complicated.

Therefore, there is a need for electrodes and a method for the production of such electrodes to measure bio signals, which mitigate one or more of the above problems.

### SUMMARY

Aspects of the present disclosure relate to a method of manufacturing a skin-compatible electrode. Preferably, the method comprises printing a conductive ink onto a flexible substrate to form an electrically conductive layer in a circuit pattern comprising an electrode pad area and a circuit lane. The method further comprises coating, printing or dispensing an adhesive composition onto the printed electrode pad area to form an adhesive interface layer in an adhesive pattern. The adhesive interface layer may be a dry film formed from the adhesive composition comprising an ionically conductive pressure sensitive adhesive composition comprising a resin, an ionic liquid, and optionally electrically conductive particles. Furthermore, a combined thickness and sizes of the flexible or stretchable substrate, the electrically conductive layer at the electrode pad area, and the adhesive interface layer, and their respective material compositions, are adapted to provide a low combined stiffness, at the electrode pad area in plane of the flexible substrate. The inventors find that electrodes having the combination of aforementioned structural, chemical, and mechanical properties may be especially suitable for long term use while allowing efficient manufacturing by printing, coating or dispensing.

### BRIEF DESCRIPTION OF DRAWINGS

These and other features, aspects, and advantages of the apparatus, systems and methods of the present disclosure will become better understood from the following description, appended claims, and accompanying drawing wherein:
FIG 1A and 1B illustrate manufacturing a skin-compatible electrode by means of a printing, coating or dispensing process;
FIG 2A illustrates a cross-section view of printing an electrically insulating pattern;
FIG 2B illustrates a cross-section view of printing, coating or dispensing an electrically insulating skin adhesive pattern;
FIG 3A illustrates a cross-section view of printing an exterior shielding pattern by printing a conductive layer;
FIG 3B illustrates a cross-section view of printing an exterior shielding pattern by printing an electrically insulating layer on top of a conductive layer;
FIG 3C illustrates a cross-section view of printing a shielding electrode by printing an electrically conductive layer on top of a shielding layer;
FIG 4A illustrates a cross-section view of printing the skin insulating pattern similar as FIG 2A but on top of a stack including an exterior shielding layer;
FIG 4B illustrates a cross-section view of printing a skin shielding pattern;
FIG 5A illustrates a plane view of a skin-compatible electrode according to a substrate cut pattern;
FIG 5B illustrates a cross-section view of the electrode as used on the skin;
FIG 5C shows a photograph of an example of electrodes manufactured as described herein;
FIG 6A illustrates a cross-section view of an electrode patch as used on the skin;
FIG 6B shows a photograph of an example of a skin-compatible patch manufactured as described herein;
FIG 7A illustrates a plane view of a grid shaped circuit pattern;
FIG 7B shows a photo of a grid of electrodes;
FIG 8A depicts impedance spectra comparing exemplary embodiments of skin-compatible electrodes 100 with differing dimensions comprising a pressure adhesive layer and comparative a gel-type electrode;
FIG 8B depicts exemplary time traces of electrical signals "E" from skin "S", recorded using a comparative gel-type electrode and a skin-compatible electrode 100 obtained according to the disclosed method. Shown traces have been obtained after 8 hours of continuous wear of said electrodes.
FIG 9A depicts exemplary time traces of electrical signals "E" from skin "S", recorded over a total time frame of 5 days recorded on day 1 with electrodes manufactured as described herein;
FIG 9B depicts exemplary time traces of electrical signals "E" from skin "S", recorded over a total time frame of 5 days recorded on day 5 with electrodes manufactured as described herein;

### DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise, it will be understood that when a connection between structures or components is described, this connection may be established directly or through intermediate structures or components unless specified otherwise.

As used herein below electrodes or single electrodes may be understood to be a single electrically conductive electrode to be placed onto skin. Typically, it is a disposable electrode comprising a lead, e.g. an external connection area, and a conductive adhesive layer on a thin film. Typically, electrodes are connectable to measurement equipment such as a Holter Monitor (e.g. Philips DigiTrak XT, GE CardioMem CM 3000) using a tab or snap connector. In the case of a tab e.g. an alligator clip can be used.

A patch may be described to comprise a plurality of electrodes connected in a specific, e.g. pre-defined, geometry. In some embodiments, the patch may be described as an ECG patch where a number of electrodes (between 3 and 5) is located in such a configuration that an ECG can be measured.

An electrode array may be understood to comprise a large number of electrodes placed in a, preferably regular, pattern. Typically, the number of electrodes in an array varies in a range between 20 to 100 electrodes.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system is constructed or operated in a particular orientation unless stated otherwise.

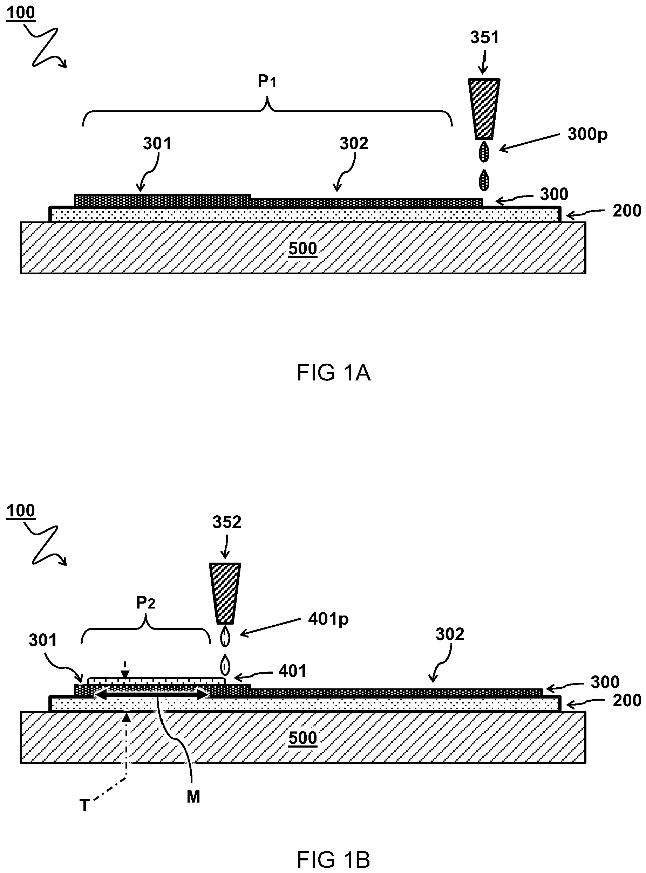

FIG 1A and 1B illustrate manufacturing a skin-compatible electrode 100 by means of a printing, coating or dispensing process;

As illustrated e.g. in FIG 1A, a preferred embodiment of manufacturing a skin-compatible electrode 100 comprises printing a conductive ink 300p onto a flexible substrate 200 which may be optionally attached to a temporary support 500. For example, the conductive ink 300p may form an electrically conductive layer 300. Preferably, the electrically conductive layer 300 is printed or dispensed according to a predefined circuit pattern P1. In some embodiments, e.g. as shown, a conductive material printer 351 is used. For example, the printer may comprise a print head, as shown. Also, any other method and systems suitable for printing, dispensing the materials and patterns as described herein may be used, in principle such as screen printing, rotary screen printing, stencil printing, flexo printing, gravure printing, Laser-Induced Forward Transfer (LIFT) printing, ink jet printing, aerosol jet printing. In some embodiments, the circuit pattern P1 comprises an electrode pad area 301. For example, the electrode pad area 301 may be used for transceiving electrical signals E via the skin S (not shown here). In other or further embodiments, the circuit pattern P1 comprises a circuit lane 302. For example, the circuit lane 302 can be electrically connected to the electrode pad area 301 for guiding the electrical signals E along the flexible substrate 200.

As illustrated e.g. in FIG 1B, another or further preferred embodiment comprises printing, coating or dispensing an adhesive composition 401p onto the printed electrode pad area 301 to form an adhesive interface layer 401. Also any other method and systems 352 suitable for coating or dispensing the materials and patterns as described herein may be used, in principle such as roll coating, gravure coating, reverse coating, roll brushing, spray coating, and air knife coating methods, immersing and curtain coating method, and extruding coating method with a die coater. Preferably, the adhesive interface layer 401 is coated, dispensed or printed in an adhesive pattern P2. As described herein, the adhesive interface layer 401 is conductive for, in use, maintaining an electrical connection for the electrical signals E between the electrode pad area 301 and the skin S (not shown here).

In a preferred embodiment, the adhesive interface layer 401 is a dry film formed from the adhesive composition 401p. As will be described and explained in further detail below, the adhesive composition 401p preferably comprises an ionically conductive pressure sensitive adhesive composition comprising a resin "R", an ionic liquid "I", and optionally electrically conductive particles "P". By using the adhesive composition 401p as specified herein good conductive contact may be provided for transducing electrical signals E from the skin S to an external device. By using the adhesive composition 401p as specified herein said conductive contact may be provided to allow prolonged use, e.g. wear, of formed electrodes 100 on - areas of skin. By using the adhesive composition 401p as specified herein good conductive contact may advantageously be provided without skin irritation compared to conventional electrodes.

In preferred embodiments, the conductive adhesive interface layer 401 has relatively low skin-electrode impedance, e.g. at 10 Hz, below one hundred mega Ohm (MΩ), preferably below fifty mega Ohm (MΩ), more preferably below ten mega Ohm (MΩ), most preferably below one mega Ohm (MΩ), for example in a range between 500 and 100 kilo Ohm (kΩ). Typically, signals with better signal to noise ratio are possible with increasingly lower impedance. In some preferred embodiments an electrically conductive adhesive layer is provided to obtain good transduction of electrical signals from skin to patch. In other or further preferred embodiments, such transduction is possible without the need of additional water-based fluids such as sweat.

Preferably, the electrode pad area 301 is compliant, e.g. having a combined stiffness less than two hundred thousand Newton per meter (200 000 N/m) more preferably below ten thousand Newton per meter (10 000 N/m). Where the stiffness is obtained by measuring the sample clamped in a tensile tester (Mark 10 ESM303) over the long side. The sample is elongated to 1% strain, preferably to 10% strain.

For example, the materials have preferably a Young's modulus "M" below two hundred mega Pascal, preferably less than one hundred mega Pascal (e.g. in plane of the flexible substrate 200) and thickness less than half a millimeter, preferably less than hundred micrometer. For example, a combined thickness "T" of the flexible substrate 200, the electrically conductive layer 300 at the electrode pad area 301, and the adhesive interface layer 401, and their respective material compositions are adapted to provide the said stretchability. In some embodiments, the flexible substrate 200 is disposed on, e.g. attached to, a (temporary) carrier substrate 500, as shown. For example, the carrier substrate 500 may provide structural stability to the flexible substrate 200 during the manufacturing process. Preferably, the carrier substrate 500 is relatively stiff, e.g. has a higher Young's modulus and thickness than the flexible substrate, e.g. higher by a factor ten or more.

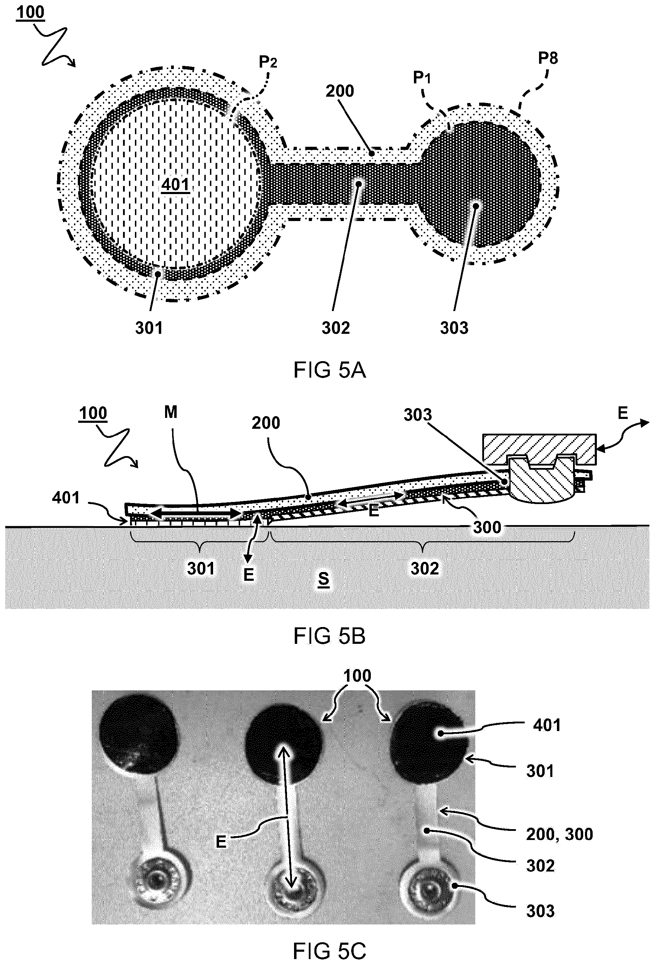

In FIG 5B an arrow marked "M" indicates the preferred stretchability of the skin-compatible electrode 100. For example, a layer thickness and material of the flexible substrate, the conductive pattern, and the conductive adhesive interface are adapted to provide a combined Young's modulus "M" below 100 MPa at the electrode pad area in plane of the flexible substrate 200. Such substrate consists preferably out of an elastomeric based (elastic) film. Preferably, the flexible substrate 200 is a medical grade polyurethane film. Such films provide good breathability and permeation to humidity. Typically examples of suitable materials may be TPU films such as those available from Delstar Technologies, Lubrizol, BASF, and Covestro. The film thickness of the flexible substrate 200 is preferably in the range between 25 and 200 µm, preferably in a range between 50 and 150 µm, e.g. 75 µm. The flexible substrate 200 is preferably stretchable, e.g. having a Young's modulus below 100 MPa. Alternatively or in addition, other types of films, e.g. rubbery films, may be used. In particular, silicone-based films may be used as substrates due to their excellent mechanical properties. Substrates based on polyether block amides, PET, PP, PEN, PEBAX and VESTAMIDE may be used as well.

The conductive ink 300p ink may be an ink or paste and typically comprises electrically conductive particles and a resin. Preferably, electrically conductive particles are selected from the group consisting of metal particles and metal nanoparticles, metal containing particles and nanoparticles, graphite particles and nanoparticles, carbon particles and nanoparticles, carbon nanowires, conductive polymer particles and nanoparticles, and mixtures thereof, more preferably selected from the group consisting of silver containing particles, silver particles, copper particles, copper containing particles, silver nanowires, copper nanowires, graphite particles, carbon particles and mixtures thereof, and even more preferably selected from graphite particles, carbon particles and mixtures thereof. Alternatively or in addition the ink may comprise conductive polymers, such as PEDOT:PSS (Poly(3,4-ethylenedioxythiophene)) or PANI (Polyaniline). Optionally ionic conductors such as Ag/AgCl or doped semiconducting materials such as Al doped ZnO, ITO or TiN particles may be used to provide conductivity. The conductive ink 300p preferably has a viscosity in the range between 0,001 and 100 Pa ·s. The thickness of formed electrode pad area 301 is preferably in a range between 1 and 50 µm, more preferably between 2 and 20 µm, e.g. 10 µm. Providing an electrode pad area 301 with a thickness below the preferred range may lead to a film with defects and/or reduced conductivity. Providing an electrode pad area 301 with a thickness above the preferred range may lead to an unacceptable increase in material cost and/or to an unacceptably high stiffness of formed conductive layer. By providing a stiffness below 200 000 N/m and preferably 10 000 N/m at the electrode pad area the formed skin-compatible electrode in use may be adapted to adhere to, and/or flex, and/or stretch along with the area of skin it is adhered to. By providing a flexible skin-compatible electrode adhesive failure between the electrode and skin may be avoided and/or comfort for the user during wear of the skin-compatible electrode may improved. Printing may be a particularly suitable technique to provide layers with a thickness in the specified ranges. Printing may be particularly suitable in depositing films in a pre-defined pattern, e.g. intricate patterns, in combination with the specified thickness ranges.

Preferably, conformal adhesion of the skin-compatible electrode 100 to the skin is provided at least for the area of the skin that is covered by the electrode pad area 401 during normal wear of the skin-compatible electrode 100 without peeling off or translocation of the electrode along the skin during wearing to avoid motion artifacts disturbing the measurement. Optionally an electrically insulating skin adhesive is applied on the electrode 100 around the electrode pad 401 and leaving an area open for connecting the tab or snap at the end of the lead 302. Preferably, once the skin-compatible electrode 100 is adhered to the skin S, the skin-compatible electrode 100 is also removable from the skin S when desired without damaging the skin S. Preferably, the skin-compatible electrode 100 is suitable for application on the skin at many locations of the body: e.g. preferably at least on the torso, for example the chest and or back, for example for electrocardiography measurements (ECG). Preferably, the skin-compatible electrode 100 is also suitable for application on skin of the head, for example, for use in electroencephalography measurements (EEG).

Preferably, the skin-compatible electrode 100 is also suitable for application on skin of the pregnant belly, for example, for use in electrohysterography measurements (EHG). Preferably, the skin-compatible electrode 100 is also suitable for application on skin over a specific muscle, for example, for use in electromyography measurements (EMG). In some preferred embodiments the electrode is also suitable for skin more prone to deformations and or flexing during wear such as locations on limbs. Preferably, good adhesion over extended periods of time is obtained enabling continuous electrical contact between skin and electrode pads to enable signal recording over prolonged periods of time: i.e. >7 days; > 30 days; without signal degradation.

In use, force may be applied to the circuit lane 302. Hence, use of conductive inks allowing stretching up to at least 1% or even at least 10% before losing essential electrical conductivity is preferred. Such inks include products such as LOCTITE ECI 1501 E&C and LOCTITE ECI 1010 E&C from Henkel. Alternatively or in addition, improved stretchability, e.g. up to 5% or even 10%, may be provided in combination with circuit pattern P1 design, e.g. a pattern suited to provide wavy or meandering circuit lanes 302.

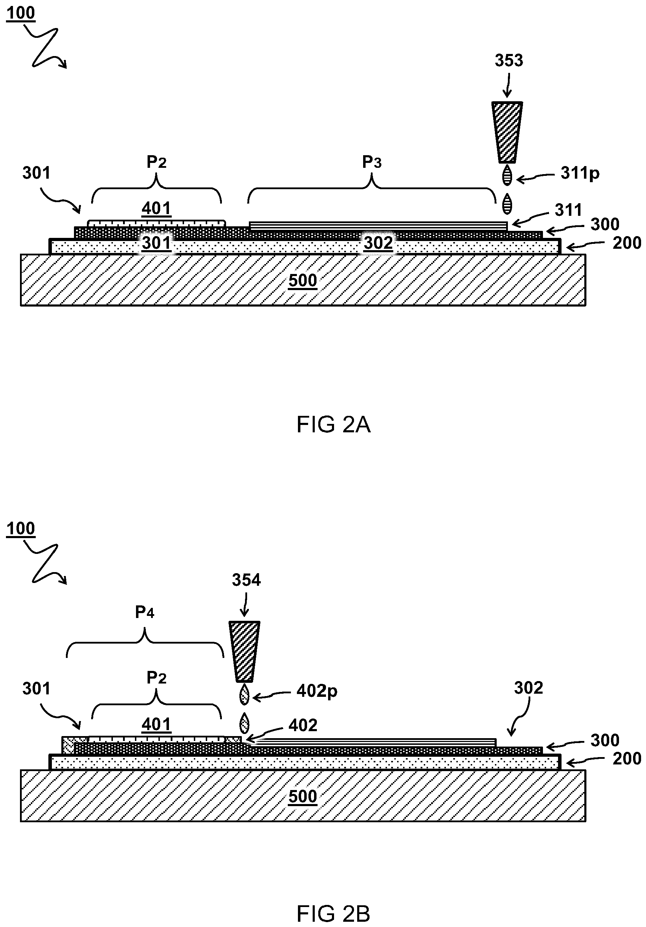

FIG 2A illustrates a cross-section view of printing a skin insulating pattern P3. In some preferred embodiments, e.g. as illustrated in FIG 2A the method preferably comprises printing an electrically insulating ink composition 311p in a skin electrically insulating pattern P3 to form a skin insulating layer 311. For example, an insulating material printer 353 is used, as shown. Preferably, the skin insulating layer 311 covers at least part of the circuit lane 302 adjacent the electrode pad area 301. By covering part of the circuit lane 302 with an electrically insulating layer, the circuit lane 302 may advantageously be electrically insulated from the skin S. Hence, unwanted interference of electrical signals resulting from contact between the skin S and the circuit lane 302 may be avoided.

FIG 2B illustrates a cross-section view of printing a dielectric skin adhesive pattern P4. For example, a skin adhesive composition printer 354 is used, as shown. In some embodiments, e.g. as illustrated in FIG 2B, the method comprises printing an electrically insulating skin adhesive composition 402p in a pattern P4 to form a dielectric skin adhesive layer 402. Preferably, the electrically insulating skin adhesive layer 402 may be provided on the flexible substrate 200 and/or on the circuit lane 302. Most preferably the electrically insulating skin adhesive layer 402 is provided at areas adjacent to the adhesive pattern P2. Advantageously, the provided electrically insulating skin adhesive layer 402 may, in use, improve the adhesion of the electrode 100 on the skin S.

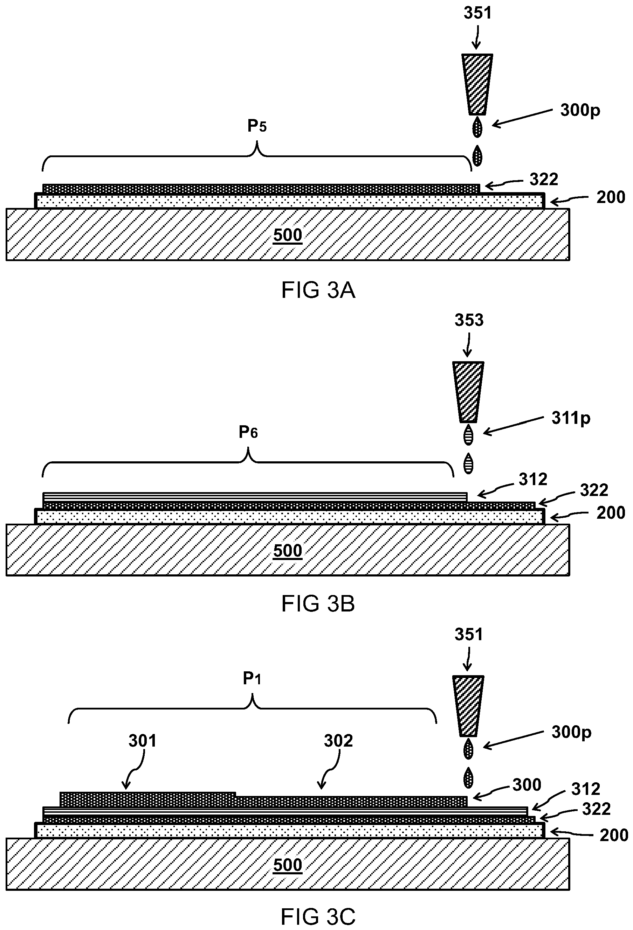

FIG 3A illustrates a cross-section view of printing an exterior shielding pattern P5. In some preferred embodiments, e.g. as shown in FIG 3A, the method comprises printing a conductive ink 300p in an exterior shielding pattern P5 to form an exterior shielding layer 322. For example, the same or similar conductive material printer 351 as in FIG 1A can be used, as shown. Preferably, the exterior shielding layer 322 is printed on the flexible substrate 200 before printing the electrically conductive layer 300. By printing the exterior shielding layer 322, the electrically conductive layer 300, in use, may be shielded from exterior electromagnetic interference. This shielding may be provided in passive or in active mode.

FIG 3B illustrates a cross-section view of printing an intermediary insulating pattern P6. For example, the same or similar electrically insulating material printer 353 as in FIG 2A can be used, as shown. In another or further preferred embodiment, e.g. as shown in FIG 2B, the method comprises printing an electrically insulating composition 311p in an intermediary electrically insulating pattern P6 to form an exterior insulating layer 312 on the exterior shielding layer 322 before printing the electrically conductive layer 300. By providing the exterior insulating layer 312 between the exterior shielding layer 322 and the electrically conductive layer 300, the exterior shielding layer 322 may be electrically insulated from the electrically conductive layer 300.

FIG 3C illustrates printing the circuit pattern P1 similar as FIG 1A, but now printed onto the intermediary insulating pattern P6 and shielding pattern P5. By printing the circuit pattern P1 onto the intermediary insulating pattern P6 and shielding pattern P5 an electrode 100 may be obtained with the properties as in FIG 1A but with the added benefit of having a shielding layer to, in use, reduce unwanted interference.

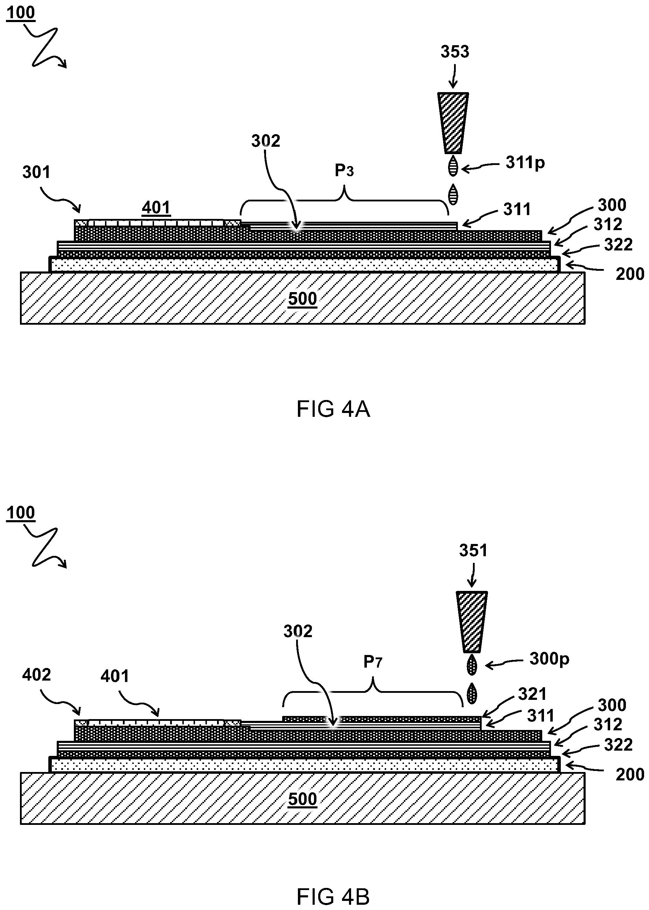

FIG 4A illustrates a cross-section view of printing the skin insulating pattern P3 similar as FIG 2A but on top of a stack including an exterior shielding layer 322.

FIG 4B illustrates a cross-section view of printing a skin shielding pattern P7. For example, the same or similar conductive material printer 351 as in FIG 1A can be used, as shown. In a preferred embodiment, the method comprises printing the conductive ink 300p in a skin shielding pattern P7 to form a skin shielding layer 321. The skin shielding layer 321 does not contact the electrode 300. Contacting the adhesive interface layer 401 may lead to the exterior shielding layer 322 functioning as an antenna, thereby introducing unwanted electromagnetic interference. Like the exterior shielding layer 322, the skin shielding layer 321 may, in use, shield the electrically conductive layer 300 from electromagnetic interference. Preferably, the skin insulating layer 311 is arranged between the skin shielding layer 321 and the circuit lane 302 for electrically insulating the skin shielding layer 321 from the electrically conductive layer 300. More preferably, the skin shielding layer 321 is electrically connected to the exterior shielding layer 322. By connecting the shielding layers, preferably along a length of the circuit lane 302 a coaxial shielding effect may be attained.

According to a further aspect, the present disclosure relates to a skin-compatible electrode 100, manufactured according to any of the manufacturing methods described herein. Preferably the electrode 100 comprises one or more aspects as described herein. It will be appreciated that by providing the electrode 100 with one or more of the elements described herein will be accompanied with the same or similar benefits as described for the corresponding elements in relation with the manufacturing methods. For example, the electrode 100 preferably comprises a flexible and/or stretchable substrate 200. More preferably, the electrode 100 comprises an electrically conductive layer 300 forming a circuit pattern P1 disposed onto the exterior insulating layer 312. For example, the circuit pattern P1 comprises an electrode pad area 301 and a circuit lane 302, as described. Further, the electrode 100 comprises an adhesive interface layer 401 formed by a dry film of an adhesive composition 401p with an ionically conductive pressure sensitive adhesive composition comprising a resin "R", an ionic liquid "I", and optionally electrically conductive particles "P". In some embodiments, the electrode 100 comprises an exterior shielding layer 322. In other or further embodiments, the electrode 100 comprises an exterior insulating layer 312. In some embodiments, the electrode 100 comprises a skin insulating layer 311. In other or further embodiments, the electrode 100 comprises a skin shielding layer 321. Preferably, the skin insulating layer 311 is arranged between the skin shielding layer 321 and the circuit lane 302. In some embodiments, the electrode 100 comprises a dielectric skin adhesive layer 402.

Preferably, the same ink 300p is used for printing the respective electrically conductive layer 300 and shielding layers 321, 322. Alternatively, different inks or other electrically conducting materials may be used. Preferably, the same dielectric composition 311p is used to form the respective insulating layers 311, 312. Alternatively, different dielectric materials may be used.

Optionally, the adhesive composition 401p is printed as solution comprising a solvent, and the adhesive interface layer 401 is a dry film formed after evaporation of the solvent. Depending on the printing method the amount of solvent in the solution and/or the type of solvent in the solutionmay be adjusted.

FIG 5A illustrates a plane view of a skin-compatible electrode 100 according to a substrate cut pattern P8. In some preferred embodiments, e.g. as shown in FIG 5A, the flexible substrate 200 is cut according to a substrate cut pattern P8. Preferably, the circuit pattern P1 forms a subset area of the substrate cut pattern P8. In other words, the conductive ink 300p is preferably printed exclusively on parts of the flexible substrate 200 which will form the electrode 100. For example, this may save expensive printing material, e.g. conductive ink comprising silver particles.

In some embodiments, e.g. as shown, the adhesive pattern P2 forms a subset area of the circuit pattern P1. For example, an intersection of the adhesive interface layer 401 and the electrically conductive layer 300 may define the effective extent of the electrode pad area 301. In other or further embodiments, the adhesive interface layer overlaps, e.g., coincides with the electrode pad area 300. By matching the area of the adhesive interface layer 401 to the electrode pad area 301, in use, charges from the skin may be effectively transferred to the skin-compatible electrode 100. By increasing a dimension of electrode pad area 301 the sensitivity of the skin-compatible electrode 100 may increase.

In some preferred embodiments the electrode pad area 301 has a dimension in a range between 5 and 75 mm, more preferably in a range between 7.5 and 50 mm, most preferably in a range between 10 and 30 mm. The thickness of formed adhesive interface layer 401 is preferably in a range between 5 and 50 µm, more preferably between 10 and 40 µm, e.g. 20 µm. Providing a conductive layer with a thickness below the preferred range may in use lead to poor adhesive properties, e.g. detaching from the skin and/or shifting (moving) of the skin-compatible electrode 100 with respect to the skin. In some embodiments, the electrode pad area 301 has an area between 20 to 4500 millimeter (mm²), preferably between 45 to 2000 square millimeter (mm²), more preferably between 60 to 1500 (mm²), and most preferably between 75 to 700 square millimeter (mm²). One benefit of providing a skin-compatible electrode 100 with a large electrode pad area 301 may be an increased signal to noise ratio. An upper limit for the electrode pad area 301 relates to the limited availability of skin area, especially in applications requiring the application of multiple skin-compatible electrodes 100, or in applications wherein the body part or person is small, for example for children.

FIG 5B illustrates a cross-section view of the electrode 100 as used on the skin "S". In some preferred embodiments, the electrically conductive layer 300 comprises an external connection area 303. For example, the external connection area 303 may be a reinforced region of the circuit lane 302. The external connection area 303 may be connected to an ExG (Electrocardiography, Electroencephalography, Electromyography, Electrohysterography) device using a rivet (as shown), electrical clamp, or any other electrical connecting means.

FIG 5C shows a photograph of an example of electrodes 100 manufactured as described herein.

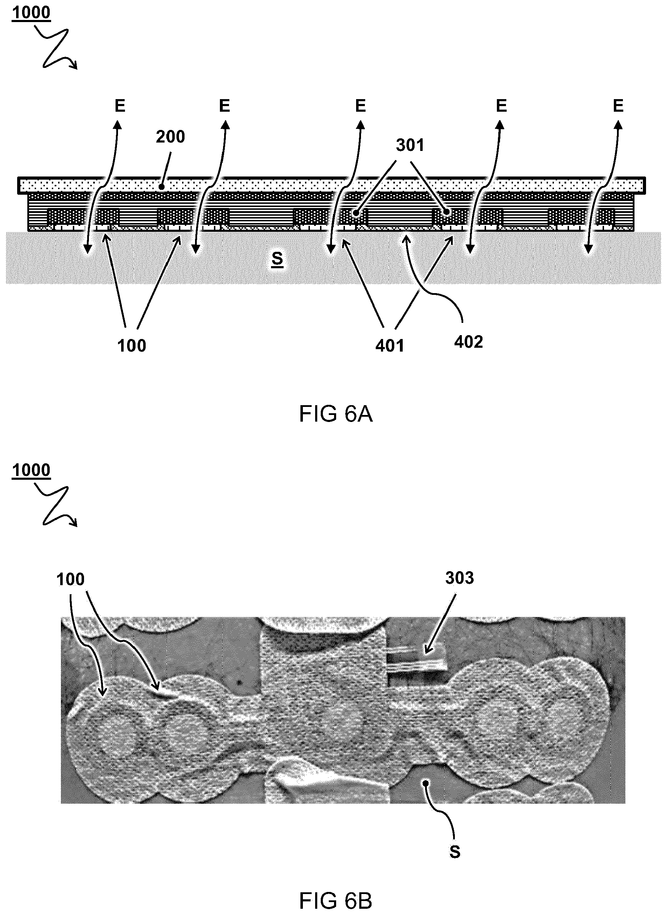

FIG 6A illustrates a cross-section view of an electrode patch 1000 as used on the skin "S". In other or further preferred embodiments, aspects of the present application relate to a method of manufacturing a skin-compatible electrode patch 1000. Preferably, the method comprises manufacturing a plurality of electrodes 100 on a common flexible substrate 200. More preferably, the electrodes 100 are arranged according to a pre-defined electrode pattern for transceiving a plurality of electrical signals "E" at respective areas of the skin "S". Most preferably, the electrode pattern comprises at least 3, e.g. 3 to 7, or 3 to 5, e.g. three electrodes 100 for measuring ECG signals via the skin "S".

In a preferred embodiment, the electrode pad areas 301 of the electrodes 100 are covered by respective adhesive interface layers 401. Preferably, the areas of the skin-compatible patch 1000 between the electrode pad areas 301 are covered by an electrically insulating skin adhesive layer 402. Providing an electrically insulating skin adhesive layer 402 may, in use, improve adhesion between skin "S" and the electrode patch 1000.

FIG 6B shows a photograph of an example of a skin-compatible patch 1000 manufactured as described herein. In some preferred embodiments, e.g. as shown in FIG 6B, the respective circuit lanes 302 of the electrodes converge at a common external connection area 303. This may allow making the connection to an external device in a fast and convenient way. It may further reduce the risk of user error in connecting the patch to the external device, especially in combination with a cable with matching connector such as a crimp connector (e.g. Nicomatic Crimpflex).

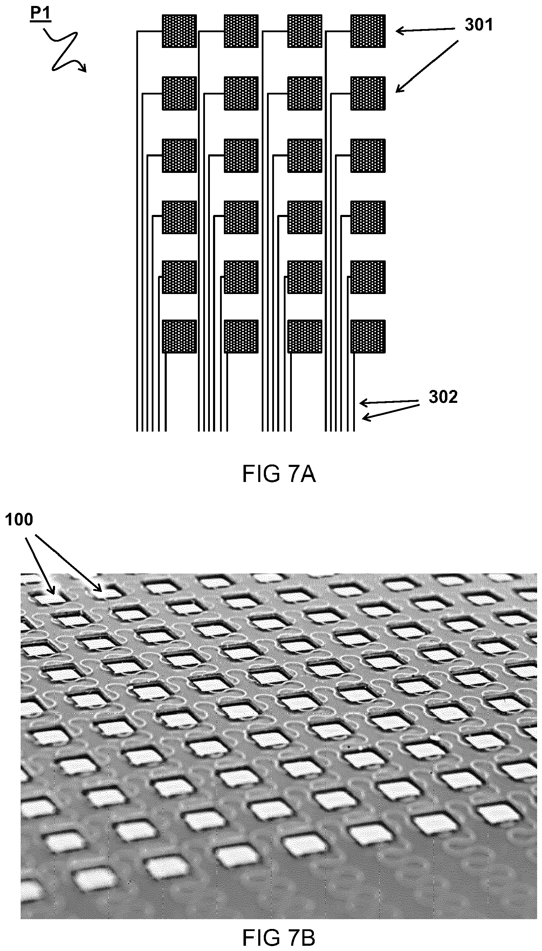

FIG 7A illustrates a plane view of a grid shaped circuit pattern P1. In some embodiments, the grid shaped circuit pattern P1 forms a two-dimensional array of spaced apart electrode pad areas 301 with respective circuit lanes 302. By providing a pattern comprising a grid of circuit shapes may allow more efficient printing of the respective layers. In some preferred embodiments, the patch comprises an array of electrode pad areas covered by dry conductive adhesive interface 401 layers for in use measuring spatial variation of electrical signals "E" over an area of the skin "S". For example, these or other patterns may be advantageous for measuring EMG signals, or the like.

FIG 7B shows a photo of a grid of electrodes 100. For example, the grid may be used in a measurement system. Generally, it will be appreciated that the various aspects as described herein may be embodied as an ExG system, e.g. Electrocardiography, Electroencephalography, Electromyography, Electrohysterography, et cetera. For example, the system comprises at least one electrode 100 and/or patch electrode patch 1000 as described herein, e.g. obtained or obtainable by any of the described methods.

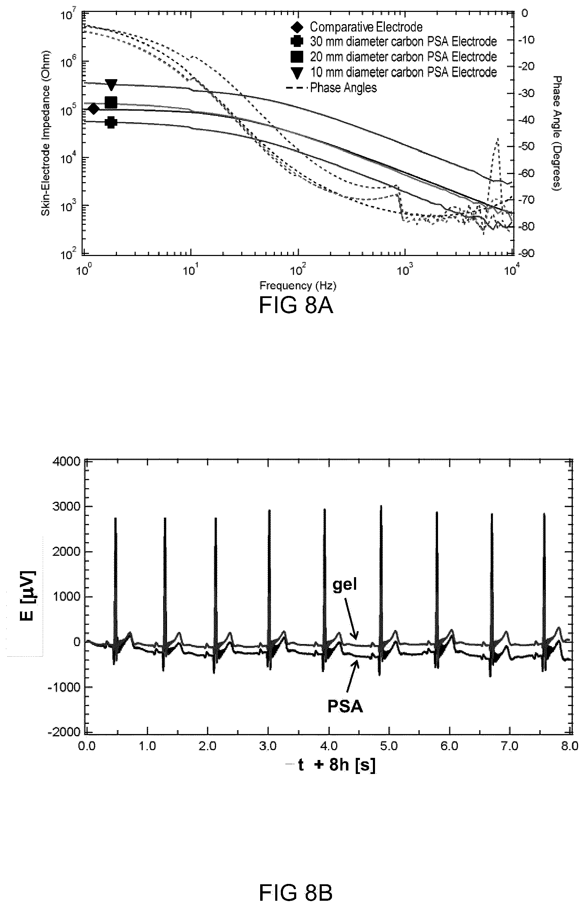

FIG 8A depicts impedance spectra comparing exemplary embodiments of skin-compatible electrodes 100 with differing dimensions comprising a pressure adhesive layer and comparative a gel-type electrode. A photo of the electrode pads of exemplary embodiments of electrodes is shown in FIG 5C. The depicted impedance spectra illustrate that the exemplary embodiments and comparative electrode show similar performance. The depicted impedance spectra further illustrate that the resistance or impedance of electrodes, as described above, may be influenced by changing the area of the electrode pad.

FIG 8B depicts exemplary time traces of electrical signals "E" from skin "S", recorded using a comparative gel-type electrode and a skin-compatible electrode 100 obtained according to the disclosed method. Shown traces have been obtained after 8 hours of continuous wear of said electrodes. The depicted time traces further illustrate that the exemplary embodiment of the skin-compatible electrode 100 has a performance similar to the comparative gel-type electrode.

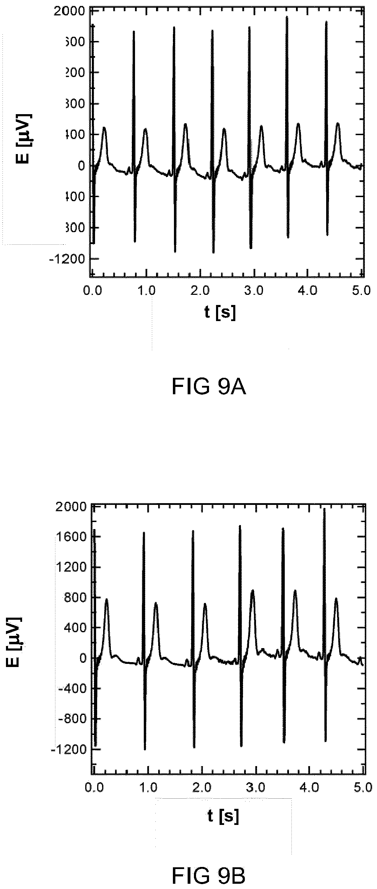

FIG 9 depicts exemplary time traces of electrical signals "E" from skin "S", recorded over a total time frame of 5 days. Said traces have been obtained during continuous wear of an exemplary embodiment of a skin-compatible electrode 100 comprising a pressure sensitive adhesive. Time traces have been obtained with 1-day intervals. FIG 9A depicts a time trace recorded on day 1, FIG 9B depicts a time trace recorded on day 5. The depicted time traces further illustrate that exemplary embodiments described herein above may be worn and used on an area of skin for prolonged periods of time without visible changes in recorded signal quality.

In the following passages preferred embodiments of the adhesive composition 401p, e.g. pressure sensitive adhesive composition (pressure sensitive adhesive) are described in more detail. Each aspect so described may be combined with any other aspect or aspects unless clearly indicated to the contrary. In particular, any feature indicated as being preferred or advantageous may be combined with any other feature or features indicated as being preferred or advantageous.

In the context of the present invention, the terms used are to be construed in accordance with the following definitions, unless a context dictates otherwise.

As used herein, the singular forms "a", "an" and "the" include both singular and plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps.

The recitation of numerical end points includes all numbers and fractions subsumed within the respective ranges, as well as the recited end points.

All percentages, parts, proportions and then like mentioned herein are based on weight unless otherwise indicated.

When an amount, a concentration or other values or parameters is/are expressed in form of a range, a preferable range, or a preferable upper limit value and a preferable lower limit value, it should be understood as that any ranges obtained by combining any upper limit or preferable value with any lower limit or preferable value are specifically disclosed, without considering whether the obtained ranges are clearly mentioned in the context.

All references cited in the present specification are hereby incorporated by reference in their entirety.

Unless otherwise defined, all terms used in disclosing the invention, including technical and scientific terms, have the meaning as commonly understood by one of the ordinary skilled in the art to which this invention belongs to. By means of further guidance, term definitions are included to better appreciate the teaching of the present invention.

The dry electrode adhesive according to the present invention is an ionically conductive pressure sensitive adhesive (PSA) with low impedance and good skin compatibility.

The ionically conductive pressure sensitive adhesive according to the present invention is preferably based on a silicone or acrylate resin, most preferably a polar solvent-based acrylic pressure sensitive adhesive with high breathability and a non-toxic, non-irritating ionic liquid leading to ionic conductivity.

The ionically conductive pressure sensitive adhesive composition 401p according to the present invention can be used as a dry film, which offers a solution for long-term monitoring of biosignals by acting as a functional contact between electrode and skin. In contrast to gel-type electrodes currently in the market it cannot dry out and it does not lead to skin irritation. Furthermore, the impedance of the PSA according to the present invention is very low without any addition of water.

More preferably, the ionically conductive pressure sensitive adhesive composition comprises a (meth)acrylate resin comprising (meth)acrylate monomer comprising OH-group (hydroxyl group) and an ionic liquid.

Most preferably, the ionically conductive pressure sensitive adhesive composition 401p according to the present invention comprises a (meth)acrylate resin comprising at least 10% of a (meth)acrylate monomer comprising OH-group by weight of the total weight of the (meth)acrylate resin.

Suitable (meth)acrylate resin for use in the present invention is preferably formed from the monomers selected from the group consisting of hydroxyethyl acrylate, hydroxypropyl acrylate, hydroxybutyl acrylate, methyl methacrylate, butyl acrylate, ethylhexyl acrylate, acrylic acid, C1-C18 alkyl (meth)acrylate, (meth)acrylamide, vinyl acetate, N-vinyl caprolactame, acrylonitrile, vinyl ether, benzyl (meth)acrylate, cyclohexyl (meth)acrylate, glycidyl (meth)acrylate and mixtures thereof, preferably formed from the monomers selected from the group consisting of hydroxyethyl acrylate, methyl methacrylate, butyl acrylate, ethylhexyl acrylate and mixtures thereof, and more preferably said (meth)acrylate resin is formed from hydroxyethyl acrylate, methyl (meth)acrylate, butyl acrylate and ethylhexyl acrylate.

Suitable commercially available (meth)acrylate resins for use in the present invention include, but not limited to Loctite DURO-TAK 222A, Loctite DURO-TAK 87-202A; Loctite DURO-TAK 87-402A; Loctite DURO-TAK 73-626A from Henkel.

The applicant has found out that a PSA comprising a (meth)acrylate resin, especially comprising at least 10% of a (meth)acrylate monomer comprising OH-group provides good impedance and electrodes do not dry out and they can be used for longer period measurement (the higher OH content increases the water vapor transmission rate of the polymer, which contributes to increased breathability and longer wear times).

Preferably content of said (meth)acrylate monomer comprising OH-group in said (meth)acrylate resin is at least 15% by weight of the total weight of the (meth)acrylate resin, preferably at least 20%, more preferably at least 25%, and most preferably at least 30%, but no more than 65%, preferably no more than 60%, more preferably no more than 55%, and most preferably no more than 50%.

When the (meth)acrylate monomer comprising OH-group in said (meth)acrylate resin is more than 65% by weight of the total weight of the (meth)acrylate resin, the higher OH-group content may negatively effect the adhesion properties.

An ionically conductive pressure sensitive adhesive composition 401p according to the present invention may comprise said (meth)acrylate resin from 5 to 80% by weight of the total weight of the composition, preferably from 15 to 75% and more preferably from 30 to 70%.

Lower (meth)acrylate resin quantity may lead to poor adhesion properties and not beneficial to film forming properties, whereas too high quantity may lead to poor conductivity.

An ionically conductive pressure sensitive adhesive composition 401p according to the present invention comprises an ionic liquid, preferably a non-toxic, non-irritating ionic liquid leading to ionic conductivity.

More specifically, an ionically conductive pressure sensitive adhesive composition 401p according to the present invention comprises an ionic liquid selected from the group consisting of imidazolium acetates, imidazolium sulfonates, imidazolium chlorides, imidazolium sulfates, imidazolium phosphates, imidazolium thiocyanates, imidazolium dicyanamides, imidazolium benzoates, imidazolium triflates, choline triflates, choline saccharinate, choline sulfamates, pyridinium acetates, pyridinium sulfonates, pyridinium chlorides, pyridinium sulfates, pyridinium phosphates, pyridinium thiocyanates, pyridinium dicyanamides, pyridinium benzoates, pyridinium triflates, pyrrolidinium acetates, pyrrolidinium sulfonates, pyrrolidinium chlorides, pyrrolidinium sulfates, pyrrolidinium phosphates, pyrrolidinium thiocyanates, pyrrolidinium dicyanamides, pyrrolidinium benzoates, pyrrolidinium triflates, phosphonium acetates, phosphonium sulfonates, phosphonium chlorides, phosphonium sulfates, phosphonium phosphates, phosphonium thiocyanates, phosphonium dicyanamides, phosphonium benzoates, phosphonium triflates, sulfonium acetates, sulfonium sulfonates, sulfonium chlorides, sulfonium sulfates, sulfonium phosphates, sulfonium thiocyanates, sulfonium dicyanamides, sulfonium benzoates, sulfonium triflates, ammonium acetates, ammonium sulfonates, ammonium chlorides, ammonium sulfates, ammonium phosphates, ammonium thiocyanates, ammonium dicyanamides, ammonium benzoates, ammonium triflates and mixtures thereof.

The term "ionic liquid" refers to a specific class of molten salts which are liquids at temperatures of 100° C or below.

Preferably, said ionic liquid "I" is selected from the group consisting of 1-ethyl-3-methylimidazolium (EMIM) acetate, 1-ethyl-3-methylimidazolium methanesulfonate, 1-ethyl-3-methylimidazolium trifluoromethanesulfonate, 1-ethyl-3-methylimidazolium chloride, 1-ethyl-3-methylimidazolium ethylsulfate, 1-ethyl-3-methylimidazolium diethylphosphate, 1-ethyl-3-methylimidazolium thiocyanate, 1-ethyl-3-methylimidazolium dicyanamide, 1-ethyl-3-methylimidazolium benzoate, choline trifluormethanesulfonate, choline saccharinate, choline acesulfamate, choline *N*-cyclohexylsulfamate, tris(2-hydroxyethyl)methylammonium methylsulfate, 1-ethyl-3-methylimidazolium tetrafluoroborate, 1-allyl-3-methylimidazolium (AMIM) bis(trifluoromethylsulfonyl)imide, 1-ethyl-3-methylimidazolium ethyl sulfate, choline acetate and mixtures thereof.

More preferably, the ionic liquid "I" is selected from the group consisting of 1-ethyl-3-methylimidazolium benzoate, 1-ethyl-3-methylimidazolium tetrafluoroborate, 1-ethyl-3-methylimidazolium methanesulfonate, 1-ethyl-3-methylimidazolium chloride, 1-ethyl-3-methylimidazolium trifluoromethanesulfonate, choline trifluoromethanesulfonate, 1-ethyl-3-methylimidazolium acetate, choline acetate, 1-ethyl-3-methylimidazolium diethylphosphate, 1-ethyl-3-methylimidazolium bis(trifluoromethylsulfonyl)imide, 1-ethyl-3-methylimidazolium ethyl sulfate, 1-ethyl-3-methylimidazolium thiocyanate, 1-ethyl-3-methylimidazolium dicyanamide, choline saccharinate, choline acesulfamate, 1-ethyl-3-methylimidazolium ethyl sulfate and mixtures thereof.

Above mentioned ionic liquids are preferred because they have good solubility in the resin, in particular (meth)acrylate resin, according to the present invention and low toxicity.

Suitable commercially available ionic liquids for use in the present invention include, but not limited to Basionics ST80, Basionics Kat1, Basionics BC01, Basionics VS11, Basionics VS03, and Efka IO 6785, all from BASF.

An ionically conductive pressure sensitive adhesive composition 401p according to the present invention may comprise an ionic liquid from 0.1 to 35% by weight of the total weight of the composition, preferably from 0.5 to 25%, and more preferably from 1 to 15%.

If the quantity of the ionic liquid "I" is too low, the adhesive may not show any ionic conductivity and the signal may be lost, whereas too high quantity may not provide improvement in signal quality but may increase the chances of skin irritation and decrease the adhesion properties.

An ionically conductive pressure sensitive adhesive composition 401p according to the present invention may further comprise an ionic conductivity promoter, preferably a non-toxic, non-irritating ionic conductivity promoter leading to additional ionic conductivity.

The ionic conductivity promoter is semi-solid or solid under room temperature and can be dissolved in the ionic liquid. It has good compatibility with the (meth)acrylate resin according to the present invention.

The ionic conductivity promoter suitable for the present invention is selected from choline chloride, choline bitartrate, choline dihydrogen citrate, choline phosphate, choline gluconate, choline fumarate, choline carbonate, choline pyrophosphate and mixture thereof.

According to the present invention, the ionically conductive pressure sensitive adhesive composition 401p according to the present invention may comprise an ionic conductivity promoter from 0.1 to 35% by weight of the total weight of the composition, preferably from 0.5 to 25%, and more preferably from 1 to 15%.

If the quantity of the ionic conductivity promoter is too low, the pressure sensitive adhesive may not show any ionic conductivity and the signal may be lost, whereas too high quantity may not provide improvement in signal quality but may increase the chances of skin irritation and decrease the adhesion properties.

To further improve conductivity, the ionically conductive pressure sensitive adhesive composition 401p according to the present invention may further comprise electrically conductive particles.

Preferably electrically conductive particles are selected from the group consisting of metal particles and metal nanoparticles, metal containing particles and nanoparticles, graphite particles and nanoparticles, carbon particles and nanoparticles, carbon nanowires, conductive polymer particles and nanoparticles, and mixtures thereof, more preferably selected from the group consisting of silver containing particles, silver particles, copper particles, copper containing particles, silver nanowires, copper nanowires, graphite particles, carbon particles and mixtures thereof, and even more preferably selected from graphite particles, carbon particles and mixtures thereof.

Graphite particles and carbon particles are preferred due the fact that they do not cause skin irritation but provide adequate conductivity.

Suitable commercially available electrically conductive particles for use in the present invention include, but not limited to Ensaco 250G, Timrex KS6 from Timcal, Printex XE2B from Necarbo, C-Nergy Super C65 from Imerys and Vulcan XC72R from Cabot.

An ionically conductive pressure sensitive adhesive composition 401p according to the present invention may comprise said electrically conductive particles from 0.1 to 35% by weight of the total weight of the composition, preferably from 0.5 to 25%, and more preferably from 1 to 15%.

If the quantity of the electrically conductive particles is too low, it may lead to poor conductivity, whereas too high quantity may lead to loss of adhesion properties.

An ionically conductive pressure sensitive adhesive composition 401p according to the present invention may further comprise a polyether polyol. Preferably, the polyether polyol is selected from polyethylene glycol (PEG), polypropylene glycol (PPG), polytetramethylene glycol (PTMG) and mixtures thereof. The applicant has found out that addition of polyether polyol is an exceptionally good host for ionic conductivity due to the open and flexible molecule chains, and therefore, has a positive impact on the impedance. The applicant has found out that already a small quantity of polyether polyol has a positive impact, which is beneficial regarding the skin compatibility of the composition.

Preferably, the polyether polyol may have a weight averaged molecular weight (Mw) from 300 to 1000 g/mol, preferably from 350 to 750 g/mol and more preferably from 380 to 420 g/mol, wherein the molecular weight is measured by gel permeation chromatography according to DIN 55672-1:2007-08 with THF as the eluent.

Suitable commercially available polyether polyols for use in the present invention include, but are not limited to Kollisolv PEG 401 from BASF.

An ionically conductive pressure sensitive adhesive composition 401p according to the present invention may comprise polyether polyol from 0.1 to 35% by weight of the total weight of the composition, preferably from 0.5 to 25% and more preferably from 1 to 15%.

Too high polyether polyol quantity may lead to loss of adhesion properties.

An ionically conductive pressure sensitive adhesive composition 401p according to the present invention may further comprise a solvent.

Suitable solvent for use in the present invention may be selected from the group consisting of water, ethyl acetate, butyl acetate, butyl diglycol, 2-butoxyethanol, ethylene glycol, diethylene glycol, propylene glycol, dipropylene glycol, methanol, isopropanol, butanol, dibasic esters, hexane, heptane, 2,4-pentadione, toluene, xylene, benzene, hexane, heptane, methyl ethyl ketone, methyl isobutyl ketone, diethylether and mixtures thereof, preferably said solvent is selected from the group consisting of ethyl acetate, butyl acetate, ethylene glycol, propylene glycol and mixtures thereof.

Suitable commercially available solvents for use in the present invention include, but not limited to ethyl acetate and ethylene glycol from Brenntag, butyl acetate from Shell Chemicals and propylene glycol from Lyondell.

An ionically conductive pressure sensitive adhesive composition 401p according to the present invention may comprise a solvent from 10 to 90% by weight of the total weight of the composition, preferably from 20 to 80%, and more preferably from 30 to 70%.

If the quantity of the solvent is too low, this may lead to processability problems due to the fact that the viscosity is too high and (meth)acrylate resin may not be fully soluble. Whereas too high quantity may lead to loss of functionality, and the viscosity of the adhesive is too low to process.

An ionically conductive pressure sensitive adhesive composition 401p according to the present invention has an impedance value below 100,000 Ohm at 1 kHz, preferably, below 20,000 Ohm at 1 kHz, wherein said impedance is measured by connecting two electrodes coated each with 25 µm of an ionic conductive pressure sensitive adhesive having a contact area of 0.25 cm².

Advantageously, the combination of the (meth)acrylate resin and the ionic liquid leads to a low impedance. The ionic liquid provides the ionic conductivity, however, if the ionic liquid "I" is not miscible with the (meth)acrylate resin, one will not see any ionic conductivity in the pressure sensitive adhesive. In the embodiment, wherein PEG is added to the composition, the additional OH-groups from the PEG make the system more polar and enhance the ionic conductivity of the ionic liquid "I" in the (meth)acrylate resin.

An ionically conductive pressure sensitive adhesive composition 401p according to the present invention has high breathability. Good breathability is obtained if the water can penetrate easily through the adhesive layer. To achieve this effect, a quite polar resin is required, in this occasion, the OH-functionalities support and improve the breathability.

Adhesive according to the present invention has a breathability value of about 4200 g/m² in 24 hours. As a comparison a standard acrylic PSA has a breathability value of about 2000 g/m² in 24 hours. The breathability is measured through a moisture vapor transmission rate (MVTR) measurement according to ASTM D1653.

The present invention also relates to a dry film formed from the ionically conductive pressure sensitive adhesive composition 401p according to the present invention.

The dry film formation can be done by coating the ionically conductive pressure sensitive adhesive composition on a supporting substrate (such as a film) and drying the film in an oven at for example 120°C for 3 minutes to remove the solvent and form a dry film of the ionically conductive pressure sensitive adhesive on the supporting substrate.

The known method used for preparing pressure-sensitive adhesive can be used. Specifically, examples include roll coating, gravure coating, reverse coating, roll brushing, spray coating, and air knife coating methods, immersing and curtain coating method, and extruding coating method with a die coater. The present invention also relates to use of an ionically conductive pressure sensitive adhesive composition 401p according to the present invention in skin applications as a contact medium as part of electrodes measuring biosignals from the skin.

The present invention also encompasses use of a dry film according to the present invention in skin applications as a contact medium as part of electrodes measuring biosignals from the skin.

For the purpose of clarity and a concise description, features are described herein as part of the same or separate embodiments, however, it will be appreciated that the scope of the invention may include embodiments having combinations of all or some of the features described. Of course, it is to be appreciated that any one of the above processes may be combined with one or more other processes to provide even further improvements in finding and matching designs and advantages. It is appreciated that this disclosure offers particular advantages to skin-compatible electrode for use on humans, and in general can be applied for any application wherein electrical signals are recorded from a surface.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. Where one claim refers to another claim, this may indicate synergetic advantage achieved by the combination of their respective features. But the mere fact that certain measures are recited in mutually different claims does not indicate that a combination of these measures cannot also be used to advantage. The present embodiments may thus include all working combinations of the claims wherein each claim can in principle refer to any preceding claim unless clearly excluded by context.

## Claims

1. A method of manufacturing a skin-compatible electrode (100), the method comprising:
- printing a conductive ink (300p) onto a flexible substrate (200) to form an electrically conductive layer (300) in a circuit pattern (P1) comprising
o an electrode pad area (301) for transceiving electrical signals (E) via the skin (S), and
o a circuit lane (302) electrically connected to the electrode pad area (301) for guiding the electrical signals (E) along the flexible substrate (200);
- printing, coating or dispensing an adhesive composition (401p) onto the printed electrode pad area (301) to form an adhesive interface layer (401) in an adhesive pattern (P2), wherein the adhesive interface layer (401) is conductive for, in use, maintaining an electrical connection for the electrical signals (E) between the electrode pad area (301) and the skin (S), wherein the adhesive interface layer (401) is a dry film formed from the adhesive composition (401p) comprising an ionically conductive pressure sensitive adhesive composition comprising a resin (R), an ionic liquid (I), and optionally electrically conductive particles (P).

2. The method according to claim 1, wherein a combined thickness (T) of the flexible substrate (200), the electrically conductive layer (300) at the electrode pad area (301), and the adhesive interface layer (401), and their respective material compositions, are adapted to provide a combined stiffness at the electrode pad area (301) in plane of the flexible substrate (200) of less than two hundred thousand Newton per meter, more preferably below ten thousand Newton per meter.

3. The method according to claim 1 or 2, wherein an electrically insulating composition (311p) is printed in a skin insulating pattern (P3) to form a skin insulating layer (311) covering at least part of the circuit lane (302) adjacent the electrode pad area (301) for, in use, electrically insulating the circuit lane (302) from the skin (S).

4. The method according to any of the preceding claims, wherein a dielectric adhesive composition (402p) is printed in an electrically insulating adhesive pattern (P4) to form an electrically insulating adhesive layer (402) on the flexible substrate (200) and/or the circuit lane (302) at areas adjacent the adhesive pattern (P2) for, in use, improving the adhesion of the electrode (100) on the skin (S).

5. The method according to any of the preceding claims, wherein the conductive ink (300p) is printed in an exterior shielding pattern (P5) to form an exterior shielding layer (322) on the flexible substrate (200) before printing the electrically conductive layer (300), for, in use, shielding the electrically conductive layer (300) from exterior electromagnetic interference.

6. The method according to any of the preceding claims, wherein the conductive ink (300p) is printed in a skin shielding pattern (P7) to form a skin shielding layer (321), for, in use, shielding the electrically conductive layer (300) from electromagnetic interference, wherein a skin insulating layer (311) is arranged between the skin shielding layer (321) and the circuit lane (302) for electrically insulating the skin shielding layer (321) from the electrically conductive layer (300).

7. The method according to any of the preceding claims, wherein the skin shielding layer (321) is electrically connected to the exterior shielding layer (322).

8. The method according to any of the preceding claims, wherein the flexible substrate (200) is cut according to a substrate cut pattern (P8), wherein the circuit pattern (P1) forms a subset area of the substrate cut pattern (P8).

9. A method of manufacturing a skin-compatible electrode patch (1000), the method comprising manufacturing comprising a plurality of electrodes (100) on a common flexible substrate (200).

10. The method according to claim 9, wherein the electrodes (100) are arranged according to a predefined electrode pattern for transceiving a plurality of electrical signals (E) at respective areas of the skin (S).

11. The method according to claim 9 or 10, wherein the electrode pattern comprises at least three electrodes (100) for measuring ECG signals via the skin (S).

12. The method according to any one or more of claims 9 to 11, wherein areas of the skin-compatible patch (1000) between the electrode pad areas (301) are covered by the electrically insulating adhesive layer (402).

13. The method according to any one or more of claims 9 to 12, wherein the respective circuit lanes (302) of the electrodes converge at a common external connection area (303).

14. The method according to any one or more of claims 9 to 13, wherein the electrode pattern forms a two-dimensional array of spaced apart electrode pad areas (301) with respective circuit lanes (302)

15. A skin-compatible electrode (100), manufactured according to any of the preceding claims, the electrode (100) comprising:
- a flexible substrate (200);
- an exterior shielding layer (322) formed by a conductive ink (300p) printed in an exterior shielding pattern (P5) onto the flexible substrate (200);
- an exterior insulating layer (312) formed by a dielectric composition (311p) printed in an intermediary insulating pattern (P6) onto the exterior shielding layer (322);
- an electrically conductive layer (300) formed by the conductive ink (300p) printed in a circuit pattern (P1) onto the exterior insulating layer (312), the circuit pattern (P1) comprising
o an electrode pad area (301) for transceiving electrical signals (E) via the skin (S), and
o a circuit lane (302) electrically connected to the electrode pad area (301) for guiding the electrical signals (E) along the flexible substrate (200);
- a skin insulating layer (311) formed by a dielectric composition (311p) printed in a skin insulating pattern (P3) onto at least part of the circuit lane (302);
- a skin shielding layer (321) formed by the conductive ink (300p) printed in a skin shielding pattern (P7), wherein the skin insulating layer (311) is arranged between the skin shielding layer (321) and the circuit lane (302);
- an adhesive interface layer (401) formed by a dry film of an adhesive composition (401p) coated or dispensed in an adhesive pattern (P2) on the electrode pad area (301), wherein the adhesive composition (401p) comprises an ionically conductive pressure sensitive adhesive composition comprising a resin (R), an ionic liquid (I), and optionally electrically conductive particles (P);
- an electrically insulating adhesive layer (402) formed by a dielectric adhesive composition (402p) printed in an electrically insulating adhesive pattern (P4) on the flexible substrate (200) and/or the circuit lane (302) at areas adjacent the adhesive pattern (P2).

16. The skin-compatible electrode (100) according to claim 15, wherein a combined thickness (T) of the flexible substrate (200), the electrically conductive layer (300) at the electrode pad area (301), and the adhesive interface layer (401), and their respective material compositions, have a combined stiffness at the electrode pad area (301) in plane of the flexible substrate (200) less than two hundred thousand Newton per meter, more preferably below ten thousand Newton per meter.

17. An ExG system comprising at least one electrode (100) according to claim 15 or 16.
